(19) Europäisches Patentamt
European Patent Office
Office européen des brevets

(11) Veröffentlichungsnummer: **0 170 842**
**B1**

(12) # EUROPÄISCHE PATENTSCHRIFT

(45) Veröffentlichungstag der Patentschrift: **13.09.89**

(51) Int. Cl.⁴: **C 07 C 103/737**, A 01 N 53/00, C 07 C 61/15

(21) Anmeldenummer: **85107613.3**

(22) Anmeldetag: **20.06.85**

(54) **N-Benzylcyclopropancarboxamid-Derivate, Verfahren zu ihrer Herstellung und Fungizide für Landwirtschaft und Gartenbau.**

(30) Priorität: **02.07.84 JP 135268/84**
**06.07.84 JP 138955/84**

(43) Veröffentlichungstag der Anmeldung: **12.02.86 Patentblatt 86/7**

(45) Bekanntmachung des Hinweises auf die Patenterteilung: **13.09.89 Patentblatt 89/37**

(84) Benannte Vertragsstaaten: **CH DE FR GB IT LI**

(56) Entgegenhaltungen:
**US-A-3 277 107**
**US-A-3 856 976**

**PATENTS ABSTRACTS OF JAPAN, Band 7, Nr. 100 (C-164) 1245 , 28. April 1983; & JP - A - 58 26847 (SUMITOMO KAGAKU KOGYO K.K.) 17.02.1983**
**PATENTS ABSTRACTS OF JAPAN, Band 7, Nr. 108 (C-165) 1253 , 11. Mai 1983; & JP - A - 58 29751 (SUMITOMO KAGAKU KOGYO K.K.) 22.02.1983**

**Die Akte enthält technische Angaben, die nach dem Eingang der Anmeldung eingereicht wurden und die nicht in dieser Patentschrift enthalten sind.**

(73) Patentinhaber: **NIHON TOKUSHU NOYAKU SEIZO K.K., No.4, 2-chome, Nihonbashi Honcho Chuo-ku, Tokyo 103 (JP)**

(72) Erfinder: **Kurahashi, Yoshio, 47- 15, Oya- machi, Hachioji- shi Tokyo (JP)**
Erfinder: **Shiokawa, Kozo, 210- 6, Shukugawara Tama- ku, Kawasaki- shi Kanagawa- ken (JP)**
Erfinder: **Kagabu, Shinzo, 432- 131- 105, Terada-machi, Hachioji- shi Tokyo (JP)**
Erfinder: **Sakawa, Shinji, 1-14- 13, Tamadaira, Hino-shi Tokyo (JP)**
Erfinder: **Moriya, Koichi, 39- 15, Namiki- cho, Hachioji- shi Tokyo (JP)**

(74) Vertreter: **Ernst, Hilmar, Dr., Bayer AG Konzernverwaltung RP Patentabteilung, D-5090 Leverkusen, Bayerwerk (DE)**

**EP 0 170 842 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft neue N-Benzylcyclopropancarboxamid-Derivate, ein Verfahren zu ihrer Herstellung und ihre Verwendung als Fungizide für Landwirtschaft und Gartenbau.

Die vor dem Einreichungsdatum der vorliegenden Anmeldung bekannte JP-OS 88555/1980 gibt an, daß N-Benzylacetamid-Derivate der folgenden allgemeinen Formel (A)

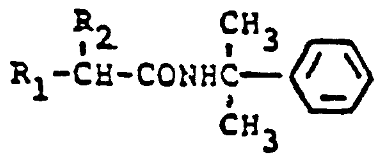

(A)

in der

$R_1$ Wasserstoff oder niederes Alkyl bezeichnet,
$R_2$ Alkyl oder Cycloalkyl bezeichnet oder
$R_1$ und $R_2$ zusammen einen Ring bilden können, herbizide Aktivität besitzen. Die Druckschrift beschreibt eine Verbindung der nachstehenden Formel:

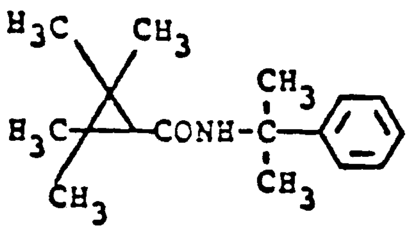

(A-1)

Die vorstehende allgemeine Formel (A) umfaßt nicht die Verbindungen der allgemeinen Formel (I), die in der vorliegenden Erfindung speziell aufgeführt sind. Darüber hinaus macht das oben erwähnte Patent-Dokument keinerlei Angaben über Fungizid-Anwendungen.

Die vor dem Einreichungsdatum der vorliegenden Anmeldung bekannte JP-OS 26847/1983 gibt an, daß N-Benzylacetamid-Derivate der folgenden allgemeinen Formel (B)

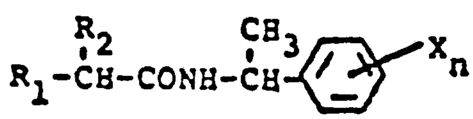

(B)

in der

$R_1$ in alpha-Stellung verzweigtes Alkyl bezeichnet,
$R_2$ Wasserstoff, niederes Alkyl oder niederes Alkenyl bezeichnet oder
$R_1$ und $R_2$ zusammen eine Alkylen-Gruppe darstellen können,
X Halogen, niederes Alkyl, niederes Alkoxy, Cyano oder Nitro bezeichnet und
n eine ganze Zahl von 1 bis 3 ist,

fungizide Aktivität in Landwirtschaft und Gartenbau besitzen. Die Druckschrift beschreibt eine Verbindung der nachstehenden Formel:

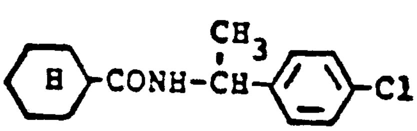

(B-1)

Die Wirkung dieser Verbindungen ist jedoch nicht auf allen Anwendungsgebieten vollständig zufriedenstellend, insbesondere dann nicht, wenn nur kleine Mengen und Konzentrationen verwendet werden.

Nunmehr wurden neue N-Benzylcyclopropancarboxamid-Derivate der nachstehenden Formel (I)

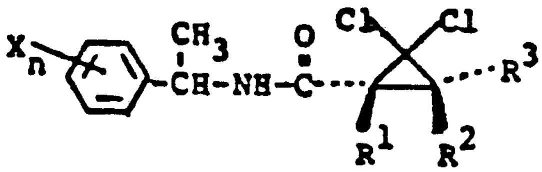

(I)

gefunden, in der

X Halogen bezeichnet,
n 1 oder 2 bezeichnet,
$R^1$ Wasserstoff, Halogen oder niederes Alkyl bezeichnet,
$R^2$ Wasserstoff, niederes Alkyl oder durch Halogen substituiertes Niederalkyl bezeichnet und
$R^3$ Wasserstoff oder niederes Alkyl bezeichnet.

Weiterhin wurde gefunden, daß die N-Benzylcyclopropancarboxamid-Derivate der Formel (I)

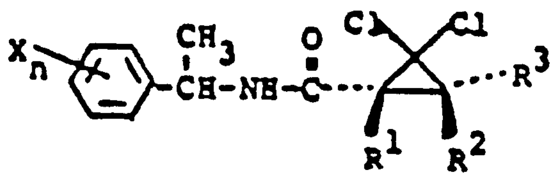

(I)

in der

X Halogen bezeichnet,
n 1 oder 2 bezeichnet,
$R^1$ Wasserstoff, Halogen oder niederes Alkyl bezeichnet,
$R^2$ Wasserstoff, niederes Alkyl oder durch Halogen substituiertes Niederalkyl bezeichnet und
$R^3$ Wasserstoff oder niederes Alkyl bezeichnet, erhalten werden, wenn Amino-Verbindungen der Formel (II)

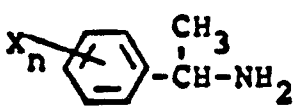

(II)

in der

X und n die im Vorstehenden angegebenen Bedeutungen haben, mit einer Verbindung der allgemeinen Formel (III)

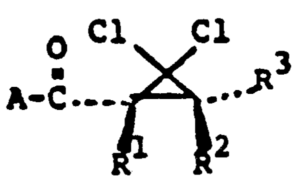

(III)

in der $R^1$, $R^2$ und $R^3$ die im Vorstehenden angegebenen Bedeutungen haben und A ein Hydroxyl oder Halogen bezeichnet, gegebenenfalls in Gegenwart eines Verdünnungsmittels und gegebenenfalls in Gegenwart eines säurebindenden Mittels umsetzt.

Die neuen N-Benzylcyclopropancarboxamid-Derivate der Formel (I) haben insbesondere fungizide Eigenschaften.

Es wurde gefunden, daß die Verbindungen gemäß der vorliegenden Erfindung eine ausgezeichnete Bekämpfungswirkung zeigen, die sich für Zwecke der Bekämpfung von Pflanzenkrankheiten besonders gut nutzen läßt. Insbesondere zeigen sie eine herausragende und ganz besonders wertvolle Wirksamkeit in bezug auf die Bekämpfung der Brusone-Krankheit bei Reis (Pyricularia oryzae). Diese Bekämpfungswirkung ist

erheblich besser als die Wirksamkeit von Verbindungen mit analoger chemischer Struktur, beispielsweise der in den bekannten Veröffentlichungen beschriebenen Verbindungen der Formeln (A - 1) und (B - 1).

Damit stellen die Verbindungen gemäß der vorliegenden Erfindung eine Bereicherung des Fachgebiets dar.

Die Formel (I) gibt eine allgemeine Definition für die N-Benzylcyclopropancarboxamid-Derivate gemäß der vorliegenden Erfindung an. Bevorzugte Verbindungen der Formel (I) sind diejenigen, in denen

X Halogen wie Fluor, Chlor, Brom und Iod bezeichnet,
n 1 oder 2 bezeichnet,
$R^1$ Wasserstoff, Halogen wie oben beispielhaft genannt oder Alkyl mit 1 bis 4 Kohlenstoff-Atomen wie Methyl, Ethyl, n-Propyl, iso-Propyl oder n-, sec-, iso- und tert-Butyl bezeichnet,
$R^2$ Wasserstoff, Alkyl wie oben beispielhaft genannt oder durch Halogen substituiertes Alkyl mit 1 bis 4 Kohlenstoff-Atomen und 1 bis 5 Halogen-Atomen bezeichnet, wobei Alkyl und Halogen diejenigen sind, die oben beispielhaft genannt sind, beispielsweise Mono- (Di- oder Tri-)chloromethyl, Mono- (Di- oder Tri-) fluoromethyl, Mono- (Di- oder Tri-)bromomethyl 1- (oder 2-)Chloro- (Bromo- oder Fluoro-)ethyl, 1,1-(1,2- oder 2,2-) Dichloro(-bromo- oder -fluoro-)ethyl und 1,1,1-(1,1,2-,1,2,2- oder 2,2,2-)Trichloro- (-bromo- oder -fluoro-)ethyl und
$R^3$ Wasserstoff oder niederes Alkyl mit 1 bis 4 Kohlenstoff-Atomen wie oben beispielhaft genannt bezeichnet.

Besonders bevorzugte Verbindungen sind diejenigen der allgemeinen Formel (I), in denen

X Fluor, Chlor oder Brom bezeichnet,
n 1 oder 2 bezeichnet,
$R^1$ Wasserstoff, Methyl, Ethyl, n-Propyl, iso-Propyl, Chlor, Brom oder Fluor bezeichnet,
$R^2$ Wasserstoff, Methyl, Ethyl, n-Propyl, iso-Propyl, n-Butyl, tert-Butyl, sec-Butyl, iso-Butyl, Chloromethyl, Bromomethyl, Trifluoromethyl, 2,2,2-Trifluoroethyl oder 2-Chloroethyl bezeichnet und
$R^3$ Wasserstoff, Methyl oder Ethyl bezeichnet.

Wenn beispielsweise 4-Chloro-α-methylbenzylamin und 1,2,2-Trichloro-3-methyl-(cis)-3-methyl-cyclopropancarbonsäurechlorid als Ausgangsstoffe eingesetzt werden, läßt sich der Reaktionsablauf des erfindungsgemäßen Verfahrens durch die folgende Gleichung darstellen:

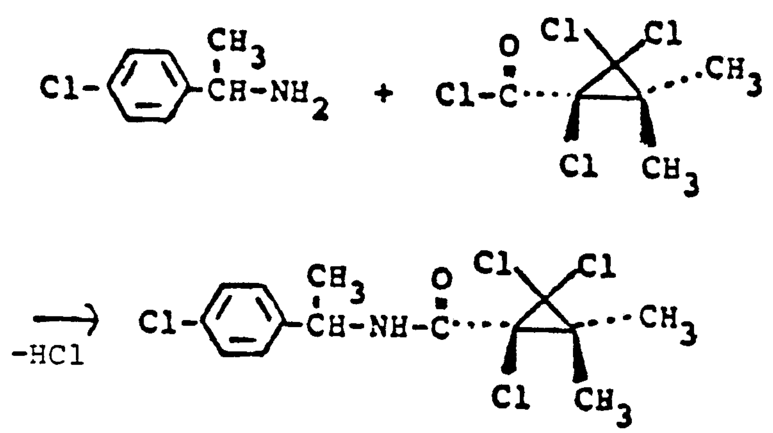

Die Formel (II) gibt eine allgemeine Definition der Amino-Verbindungen, die als Ausgangstoffe für die Durchführung des Verfahrens gemäß der vorliegenden Erfindung benötigt werden. In dieser Formel haben X und n die gleichen Bedeutungen, wie sie bei der Beschreibung der Verbindungen der Formel (I) angegeben wurden.

Die Verbindungen der Formel (II) sind bekannt. Zu speziellen Beispielen für die Ausgangsverbindungen der Formel (II) zählen beispielsweise 4-Chloro-, 4-Fluoro-, 4-Bromo-, 2-Chloro-, 3,4-Dichloro- und 2,6-Dichloro-α-methylbenzylamin.

Die Formel (III) gibt eine allgemeine Definition der Cyclopropancarbonsäure-Derivate, die als Ausgangstoffe für die Durchführung des Verfahrens gemäß der vorliegenden Erfindung benötigt werden. In dieser Formel haben $R^1$, $R^2$ und $R^3$ die gleichen Bedeutungen, wie sie bei der Beschreibung der Verbindungen der Formel (I) angegeben wurden, und A bezeichnet vorzugsweise Hydroxy oder Halogen wie Chlor und Brom.

Zu speziellen Beispielen für die Verbindungen der allgemeinen Formel (III), die in gleicher Weise Ausgangsstoffe sind, zählen 2,2-Dichloro-(trans)-3-chloro-methyl-(cis)-3-methylcyclopropancarbonsäure, 1,2,2-Trichloro-3,3-dimethylcyclopropancarbonsäure, 1,2,2-Trichloro-3,3-diethylcyclopropancarbonsäure, 2,2-Dichloro-(trans)-3-bromomethyl-(cis)-3-methylcyclopropancarbonsäure, 2,2-Dichloro-(trans)-3-trifluoromethyl-(cis)-3-methylcyclopropancarbonsäure, 1-Bromo-2,2-dichloro-3,3-dimethylcyclopropancarbonsäure, 2,2-Dichloro-1-fluoro-3,3-dimethylcyclopropancarbonsäure, 2,2-Dichloro-(trans)-3-(2,2,2-trifluoroethyl)-(cis)-3-methylcyclopropancarbonsäure sowie 2,2-Dichloro -(trans)-3-(2-chloroethyl)-(cis)-3-methylcyclopropancarbonsäure, 2,2-Dichloro-1-methylcyclopropancarbonsäure, 2,2-Dichloro-1-methylcyclopropancarbonsäure, 2,2-Dichloro-(trans)-3-methylcyclopropancarbonsäure, 2,2-Dichloro-1-methyl-(trans)-3-methylcyclopropancarbonsäure, 2,2-Dichloro-1-ethyl-(trans)-3-ethylcyclopropancarbonsäure, 2,2-Dichloro-3,3-dimethylcyclopropancarbonsäure, 2,2-Dichloro-3,3-diethylcyclopropancarbonsäure und 2,2-Dichloro-1,3,3-trimethylcyclopropancarbonsäure. Halogenide, etwa die Chloride und Bromide, dieser Carbonsäuren können ebenfalls als Beispiele genannt werden.

Einige der Cyclopropancarbonsäure-Derivate der Formel (III) sind bekannt.

Verbindungen, die noch nicht bekannt sind, sind diejenigen der Formel (IIIa)

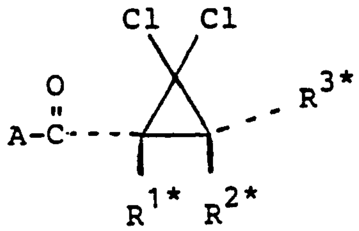

(IIIa)

in der

$R^{1*}$ Wasserstoff, Halogen oder Alkyl bezeichnet und

$R^{2*}$ niederes Alkyl oder durch Halogen substituiertes Niederalkyl bezeichnet, mit der Maßgabe, daß, wenn $R^{1*}$ ein Wasserstoff-Atom oder Alkyl ist, $R^{2*}$ durch Halogen substituiertes Niederalkyl bezeichnet,

$R^{3*}$ niederes Alkyl bezeichnet und

A Hydroxyl oder Halogen wie Chlor und Brom bezeichnet.

Bevorzugte Verbindungen der Formel (IIIa) sind die, in denen

$R^{1*}$ für Wasserstoff, Halogen, wie Fluor, Chlor, Brom und Jod, oder für Alkyl mit 1 bis 4 Kohlenstoffatomen, wie z. B. Methyl, Ethyl, n- und iso-Propyl, n-, sec., iso- oder tert. Butyl, steht,

$R^{2*}$ für Alkyl wie oben angegeben oder für Halogenalkyl mit 1 bis 4 Kohlenstoffatomen und 1 bis 5 Halogenatomen steht, wobei Alkyl und Halogen die oben angegebene Bedeutung haben, beispielhaft seien genannt: Mono-(Di- oder Tri)-chlormethyl, Mono-(Di- oder Tri-)fluormethyl, Mono-(Di- oder Tri-)brommethyl, 1-(oder 2-) Chlor-(Brom- oder Fluor-)ethyl, 1,1-(1,2- oder 2,2-(Dichlor(-brom- oder -fluor-)ethyl und 1,1,1-(1,1,2-, 1,2,2- oder 2,2,2-)Trichlor-(-brom-oder -fluor-)ethyl, mit der Maßgabe, daß wenn $R^{1*}$ Wasserstoff oder Alkyl ist, $R^{2*}$ für Halogenalkyl steht,

$R^{3*}$ für Niederalkyl mit 1 bis 4 Kohlenstoffatomen, wie oben ausgeführt, steht und

A für Hydroxyl oder Halogen, wie Chlor und Brom, steht.

Besonders bevorzugt sind die Verbindungen der Formel (IIIa), in der

$R^{1*}$ für Wasserstoff, Methyl, Ethyl, n-Propyl, iso-Propyl, Chlor, Brom oder Fluor steht,

$R^{2*}$ für Methyl, Ethyl, n-Propyl, iso-Propyl, n-Butyl, sec.-Butyl, iso-Butyl, tert.-Butyl, Chlormethyl, Brommethyl, Trifluormethyl, 2,2,2-Trifluorethyl oder 2-Chlorethyl steht, mit der Maßgabe, wenn $R^{1*}$ für Wasserstoff oder Alkyl steht, $R^{2*}$ für Halogenalkyl, wie angegeben, steht,

$R^{3*}$ für Methyl oder Ethyl steht und

A die oben gegebene Bedeutung hat.

Die Verbindungen der allgemeinen Formel (IIIa) gemäß der vorliegenden Erfindung können in einfacher Weise hergestellt werden, beispielsweise mittels der nachstehenden Verfahren:

**Verfahren i)**

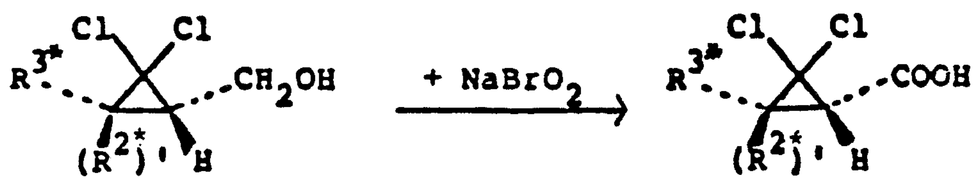


(IV) (III')

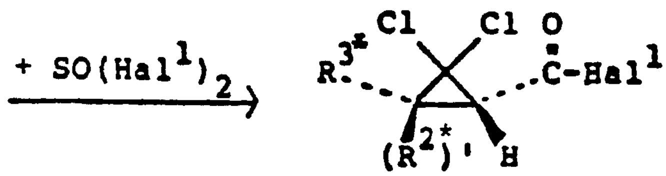


(III")

In den Formeln bezeichnet $(R^{2*})'$ durch Halogen substituiertes Niederalkyl, $Hal^1$ bezeichnet ein Halogen, und $R^{3*}$ hat die im Vorstehenden angegebene Bedeutung.

**Verfahren ii)**

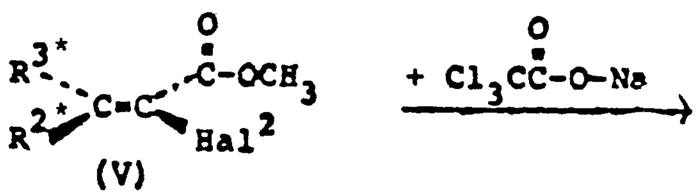

(V)

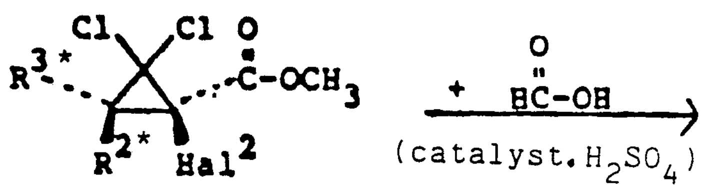


(VI)

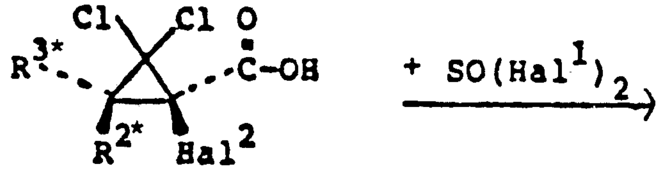


(III"')

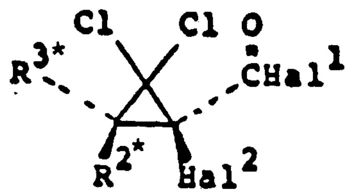

(III"")

In den Formeln haben $R^{2*}$, $R^{3*}$ und $Hal^1$ die im Vorstehenden angegebenen Bedeutungen, und $Hal^2$ bezeichnet ein Halogen-Atom.

Zu speziellen Beispielen für die Verbindungen der allgemeinen Formel (IV), die Ausgangsstoffe für das Verfahren i) sind, zählen 2,2-Dichloro-(trans)-3-chloromethyl-(cis)-3-methylcyclopropanmethanol, 2,2-Dichloro-(trans)-3-bromomethyl -(cis)-3-methylcyclopropanmethanol, 2,2-Dichloro -(trans)-3-trifluoromethyl-(cis)-3-methylcyclopropanmethanol, 2,2-Dichloro-(trans) -3-(2,2,2-trifluoroethyl)-(cis)-3-methylcyclopropanmethanol und 2,2-Dichloro-(trans)-3-(2-chloroethyl)-(cis)-3-methylcyclopropanmethanol.

Spezielle Beispiele für die Verbindungen der allgemeinen Formel (V), die Ausgangsstoffe für das Verfahren ii) sind, sind Methyl-2-chlorocrotonat, Methyl-2-bromocrotonat, Methyl-2-fluorocrotonat und Methyl-2-chloro-3-ethyl-2-pentennoat. An Stelle der Methylester der allgemeinen Formel (V) können auch andere entsprechende Alkylester genannt werden.

An Stelle des Natriumtrichloroacetats, das in dem Verfahren ii) mit der Verbindung der allgemeinen Formel (V) umzusetzen ist, können 50-proz. Natriumhydroxid und ein Phasentransfer-Katalysator wie Triethylbenzylammoniumchlorid in Chloroform verwendet werden.

Die Verfahren i) und ii) werden anhand der nachfolgenden typischen Beispiele im einzelnen beschrieben.

i - 1

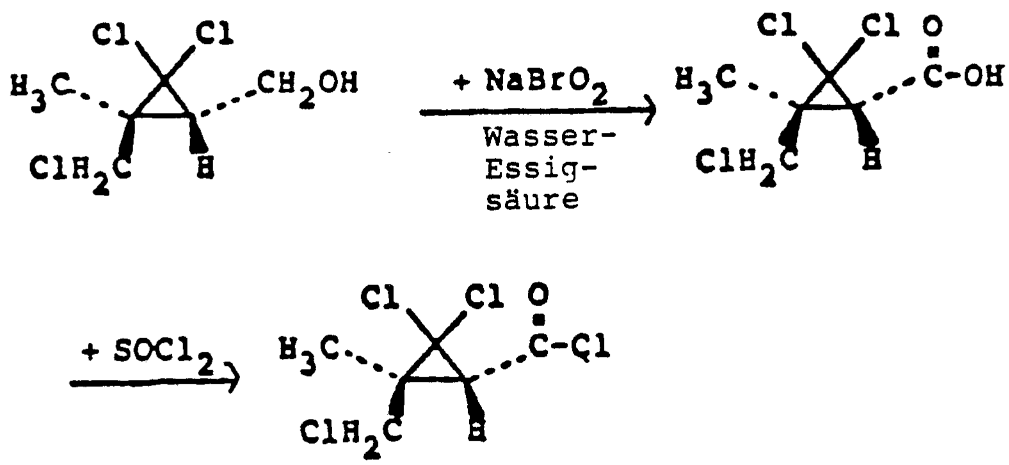


ii - 1

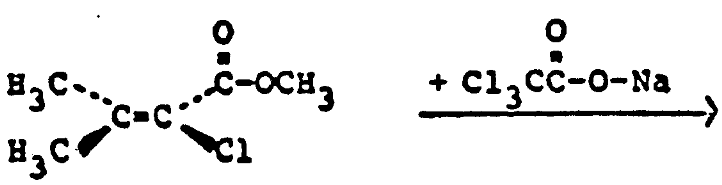

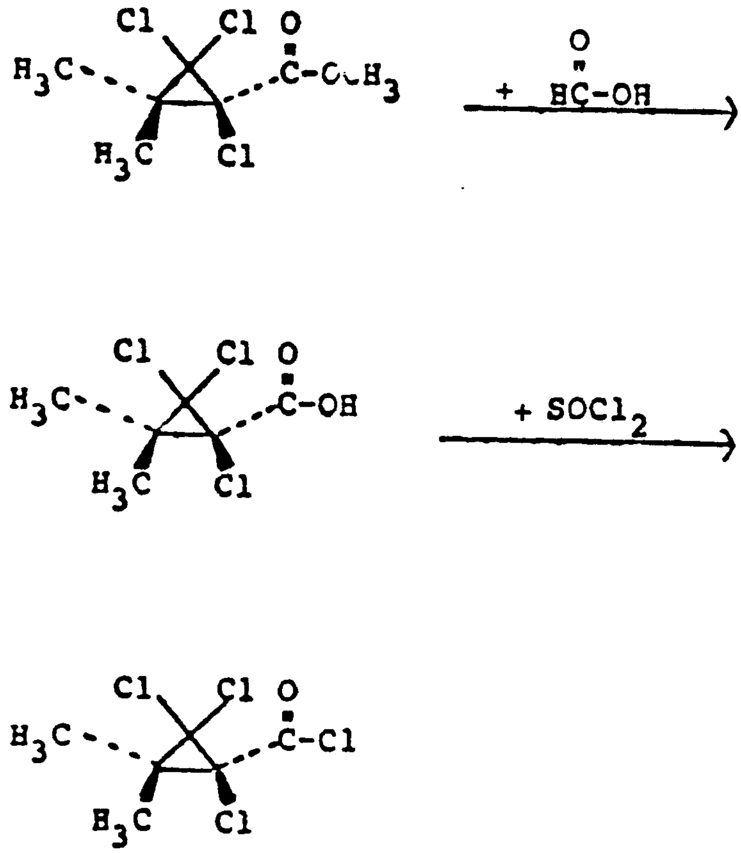


Zur Durchführung der vorstehenden Verfahren können die gleichen inerten Lösungsmittel oder Verdünnungsmittel verwendet werden, wie sie im Folgenden aufgeführt werden und wie sie auch für die Herstellung der Produkte der Formel (I) eingesetzt werden.

Diese Verfahren i) und ii) können innerhalb eines breiten Temperaturbereichs durchgeführt werden, beispielsweise bei einer Temperatur von etwa -20°C und dem Siedepunkt der Mischung, vorzugsweise bei einer Temperatur zwischen etwa 0°C und etwa 100°C. Vorzugsweise wird die Reaktion unter Atmosphärendruck durchgeführt, jedoch ist es ebenfalls möglich, bei erhöhtem oder vermindertem Druck zu arbeiten.

Das Verfahren gemäß der vorliegenden Erfindung kann zweckmäßigerweise in Gegenwart eines Lösungsmittels oder Verdünnungsmittels durchgeführt werden. Zu diesem Zweck können alle inerten Lösungsmittel oder Verdünnungsmittel verwendet werden.

Beispiele für solche Lösungsmittel oder Verdünnungsmittel umfassen Wasser; aliphatische, alicyclische und aromatische Kohlenwasserstoffe (die gegebenenfalls chloriert sein können), wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol, Ether wie Diethylether, Methylethylether, Di-isopropylether, Dibutylether, Propylenoxid, Dioxan und Tetrahydrofuran, Ketone wie Aceton, Methylethylketon, Methylisopropylketon und Methylisobutylketon, Nitrile wie Acetonitril, Propionitril und Acrylnitril, Ester wie Ethylacetat und Amylacetat, Säureamide wie Dimethylformamid und Dimethylacetamid, Sulfone und Sulfoxide wie Dimethylsulfoxid und Sulfolan.

Die obige Reaktion kann in Gegenwart eines säurebindenden Mittels durchgeführt werden. Beispiele für das säurebindende Mittel umfassen die Hydroxide, Carbonate, Hydrogencarbonate und Alkoholate von Alkalimetallen sowie tertiäre Amine wie Triethylamin, Diethylanilin und Pyridin, die sämtlich allgemein verwendet werden.

Wenn in dem vorstehenden Verfahren als Ausgangsmaterial der allgemeinen Formel (III) die Carbonsäure verwendet wird, kann ein dehydrierend-kondensierendes Mittel eingesetzt werden. Ein Beispiel ist N,N'-Dicyclohexylcarbodiimid.

Das Verfahren gemäß der vorliegenden Erfindung kann in einem weiten Temperaturbereich durchgeführt

werden. Im allgemeinen kann es bei einer Temperatur zwischen etwa -20°C und dem Siedepunkt der Mischung, vorzugsweise zwischen etwa 0°C und etwa 100°C, durchgeführt werden. Die Reaktion wird zweckmäßigerweise unter normalem Atmosphärendruck durchgeführt. Es ist jedoch auch möglich, unter erhöhtem oder vermindertem Druck zu arbeiten.

Die Verbindungen der vorliegenden Erfindung können sicher eingesetzt werden, ohne Phytotoxizität gegen Pflanzen zu zeigen. Sie zeigen eine hervorragende Bekämpfungswirkung außer gegen Reis-Schalenmehltau gegenüber einem breiten Bereich von Pflanzenerkrankung und besitzen eine ausgezeichnete Residualwirkung.

Dementsprechend lassen sie sich vorteilhaft und günstig zur Bekämpfung eines breiten Bereichs von Pflanzenerkrankungen verwenden.

Das fungizide Spektrum der Verbindungen der vorliegenden Erfindung zeigt, daß sie in wirksamer Weise gegen Pflanzenkrankheiten verwendet werden können, die durch Archimycetes, Phycomycetes, Ascomycetes, Basidiomycetes und Fungi imperfecti sowie Bakterien verursacht werden.

Typische Beispiele, die in das fungizide Spektrum der Verbindungen gemäß der vorliegenden Erfindung fallen, sind die Brusone-Krankheit von Reis (Pyricularia oryzae) und die Brennfleckenkrankheit der Gurken, Melonen und dergleichen (Colletotrichum lagenarium). Der Bereich des fungiziden Spektrums ist jedoch nicht hierauf beschränkt.

Als Fungizide für Landwirtschaft und Gartenbau können die Verbindungen der Formel (I) gemäß der vorliegenden Erfindung unmittelbar nach Verdünnen mit Wasser oder aber in Form verschiedener Formulierungen eingesetzt werden, wie sie unter Verwendung landwirtschaftlich unbedenklicher Hilfsstoffe durch Verfahren gewonnen werden, die bei der praktischen Herstellung von Agrochemikalien allgemein angewandt werden. Im praktischen Einsatz werden diese Präparate entweder unmittelbar oder nach dem Verdünnen mit Wasser auf die gewünschte Konzentration zur Anwendung gebracht.

Die landwirtschaftlich unbedenklichen Hilfsstoffe, auf die hier Bezug genommen wird, umfassen beispielsweise Verdünnungsmittel (Lösungsmittel, Streckmittel, Träger), oberflächenaktive Mittel (Lösungsvermittler, Emulgatoren, Dispergiermittel, Netzmittel), Stabilisatoren, Haftmittel, Aerosol-Treibmittel und synergistische Mittel.

Beispiele für die Lösungsmittel sind Wasser und organische Lösungsmittel, beispielsweise Kohlenwasserstoffe [z. B. n-Hexan, Petrolether, Naphtha, Erdöl-Fraktionen (z. B. Paraffinwachse, Kerosin, Leichtöle, Mittelöle und Schweröle), Benzol, Toluol und Xylol], halogenierte Kohlenwasserstoffe (z. B. Methylenchlorid, Kohlenstofftetrachlorid, Ethylenchlorid, Ethylendibromid, Chlorbenzol und Chloroform), Alkohole (z. B. Methanol, Ethanol, Propanol und Ethylenglycol), Ether (z. B. Diethylether, Ethylenoxid und Dioxan), Alkoholether (z. B. Ethylenglycol-monomethylether), Ketone (z. B. Aceton und Isophoron), Ester (z. B. Ethylacetat und Amylacetat), Amide (z. B. Dimethylformamid und Dimethylacetamid) und Sulfoxide (z. B. Dimethylsulfoxid).

Beispiele für die Streckmittel oder Träger umfassen anorganische Pulver, beispielsweise Schwefel, Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z. B. Pyrophyllit, Talkum, Montmorillonit, Beidellit, Vermikulit, Kaolinit und Glimmer), pflanzliche Pulver wie Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für oberflächenaktive Mittel umfassen anionische oberflächenaktive Mittel wie Alkylsulfonsäureester (z. B. Natriumlaurylsulfat), Akrylsulfonsäuren (z. B. Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z. B. Laurylamin, Stearyltrimethylammoniumchlorid und Alkyldimethylbenzylammoniumchlorid) und Polyoxyethylenalkylamine, nicht-ionische oberflächenaktive Mittel wie Polyoxyethylenglycolether (z. B. Polyoxyethylenalkylarylether und deren Kondensationsprodukte), Polyoxyethylenglycolester (z. B. Polyoxyethylenfettsäureester) und Ester mehrwertiger Alkohole (z. B. Polyoxyethylensorbitan-monolaurat) sowie amphotere oberflächenaktive Mittel.

Beispiele für andere Hilfsstoffe umfassen Stabilisatoren, Haftmittel (z. B. landwirtschaftliche Seifen, Casein-Kalk, Natriumalginat, Polyvinylalkohol, Haftmittel vom Vinylacetat-Typ und Acryl-Haftmittel), Aerosol-Treibmittel (z. B. Trichlorofluoromethan, Dichlorofluoromethan, 1,2,2-Trichloro-1,1,2-trifluoroethan, Chlorbenzol, verflüssigtes Erdgas und niedere Ether), die Verbrennung steuernde Mittel für Räucherpräparate (z. B. Nitrite, Zink-Pulver und Dicyandiamid), Sauerstoff abgebende Mittel (z. B. Chlorate), wirkungsverlängernde Mittel, Dispersions-Stabilisatoren [z. B. Casein, Tragant, Carboxymethylcellulose (CMC) und Polyvinylalkohol (PVA)] und synergistische Mittel.

Die Verbindungen gemäß der vorliegenden Erfindung können mittels allgemein bei der Herstellung von Agrochemikalien angewandter Methoden zu verschiedenartigen Präparaten formuliert werden. Erläuternde Beispiele für solche Anwendungsformen sind emulgierbare Konzentrate, Öl-Präparate, benetzbare Pulver, lösliche Pulver, Suspensionen, Stäubemittel, Granulat, Pulverpräparate, Räuchermittel, Tabletten, Aerosole, Pasten und Kapseln.

Die land- und gartenwirtschaftlichen Fungizide gemäß der vorliegenden Erfindung können etwa 0,1 bis etwa 95 Gew.-%, vorzugsweise etwa 0,5 bis etwa 90 Gew.-% des vorerwähnten aktiven Wirkstoffes enthalten.

Für den praktischen Gebrauch beträgt die geeignete Menge der aktiven Verbindung in den vorgenannten Mitteln in ihren verschiedenen Formen und gebrauchsfertigen Präparaten beispielsweise etwa 0,0001 bis etwa 20 Gew.-%, vorzugsweise etwa 0,005 bis etwa 10 Gew.-%.

Der Gehalt des Wirkstoffs kann in geeigneter Weise je nach der Form des Präparats oder Mittels, dem

Verfahren, Zweck, der Zeit und dem Ort seiner Anwendung, dem Zustand des Auftretens einer Pflanzenkrankheit etc. variiert werden.

Erforderlichenfalls können die Verbindungen gemäß der vorliegenden Erfindung weiterhin auch in Kombination mit anderen Agrochemikalien verwendet werden, beispielsweise mit Insektiziden, anderen Fungiziden, Mitiziden, Nematiziden, Anti-Virus-Mitteln, Herbiziden, Pflanzen-Wachstumsregulatoren und Lockstoffen [Organophosphat-Verbindungen, Carbamat-Verbindungen, Dithio(oder thiol)carbamat-Verbindungen, organischen Chlor-Verbindungen, Dinitro-Verbindungen, organischen Schwefel- oder Metall-Verbindungen, Antibiotika, substituierten Diphenylether-Verbindungen, Harnstoff-Verbindungen und Triazin-Verbindungen] und/oder Düngemitteln.

Die den im Vorstehenden bezeichneten Wirkstoff enthaltenden verschiedenartigen Mittel und gebrauchsfertigen Präparate können mittels verschiedener Verfahren zur Anwendung gebracht werden, wie sie allgemein für das Aufbringen von Agrochemikalien gebräuchlich sind, beispielsweise durch Sprühen (Versprühen von Flüssigkeiten, Vernebeln, Zerstäuben, Stäuben, Streuen von Granulat, Wasser-Oberflächenbehandlung, Gießen etc.), Räuchern, Bodenbehandlung (Vermischen mit dem Boden, Streuen, Bedampfen, Gießen etc.), Oberflächenbehandlung (Beschichten, Bändern, Bestäuben, Bedecken etc.) und Eintauchen. Sie können auch mittels des sogenannten Ultra-Low-Volume-Sprühverfahrens aufgebracht werden. Nach diesem Verfahren kann der Wirkstoff sogar in einer Konzentration von 100 % zur Anwendung gelangen.

Die Aufwandmengen pro Flächeneinheit betragen beispielsweise etwa 0,03 bis etwa 10 kg/ha, vorzugsweise etwa 0,3 bis etwa 6 kg/ha. In besonders gelagerten Fällen können oder sollten sogar die Aufwandmengen außerhalb des angegebenen Bereichs liegen.

Gemäß der vorliegenden Erfindung kann ein fungizides Mittel für Landwirtschaft und Gartenbau verfügbar gemacht werden, das eine Verbindung der allgemeinen Formel (I) als Wirkstoff und ein Verdünnungsmittel (ein Lösungsmittel und/oder ein Streckmittel und/oder einen Träger) und/ oder ein oberflächenaktives Mittel und, sofern weiter erforderlich, einen Stabilisator, ein Haftmittel, ein synergistisches Mittel etc. enthält.

Die vorliegende Erfindung stellt ebenfalls ein Verfahren zur Bekämpfung von Erkrankungen von Nutzpflanzen zur Verfügung, bei dem auf Pathogene und/oder den Ort ihres Auftretens und/oder den Ort des Auftretens der Erkrankungen von Nutzpflanzen eine Verbindung der Formel (I) allein oder im Gemisch mit einem Verdünnungsmittel (einem Lösungsmittel und/oder einem Streckmittel und/oder einem Träger) und/oder einem oberflächenaktiven Mittel und, sofern weiter erforderlich, einem Stabilisator, einem Haftmittel, einem synergistischen Mittel etc. aufgebracht wird.

Die folgenden Beispiele erläutern die vorliegende Erfindung im einzelnen. Es sei jedoch darauf hingewiesen, daß die vorliegende Erfindung nicht allein auf diese speziellen Beispiele beschränkt ist.

**Synthese von Zwischenprodukten**

Beispiel a)

8 g 2,2-Dichloro-(trans)-3-chloromethyl-(cis)-3-methylcyclopropanmethanol wurden in einem Mischlösungsmittel aus Essigsäure (30 ml) und Wasser (6 ml) suspendiert, und 16 g Natriumbromit-hydrat (etwa 60 % als Natriumbromit) wurden bei Raumtemperatur hinzugefügt. Nach der Zugabe wurde die Reaktionstemperatur auf 40°C gesteigert. Nachdem die Reaktion insgesamt während eines Zeitraums von 8 h durchgeführt worden war, wurde die Reaktionsmischung in Eiswasser gegossen, mit verdünnter Salzsäure auf pH 5 eingestellt und mit Ether extrahiert. Die etherische Schicht wurde mit einer wäßrigen Lösung von Natriumthiosulfat gewaschen und über wasserfreiem Natriumsulfat getrocknet. Der Ether wurde unter vermindertem Druck abgedampft, und der Rückstand wurde aus Hexan umkristallisiert, wonach die gewünschte 2,2-Dichloro-(trans)-3-chloromethyl-(cis)-3-methylcyclopropancarbonsäure (3,2 g) der folgenden Formel erhalten wurde; Schmp. 118-120°C.

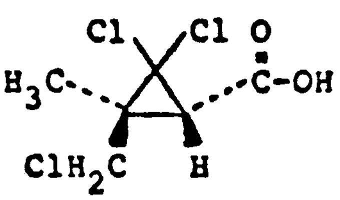

(Verbindung Nr. III-a)

Beispiel b)

Die in Beispiel a) synthetisierte Verbindung Nr. III-a (3,0 g) wurde mit Thionylchlorid (1,0 ml) bei 60°C umgesetzt. Destillation der Reaktionsmischung unter vermindertem Druck lieferte das gewünschte 2,2-Dichloro-(trans)-3-chloromethyl-(cis)-3-methylcyclopropancarbonsäurechlorid (2,8 g) der folgenden Formel; Sdp. 105 - 107°C/26,7 mbar (20 mmHg).

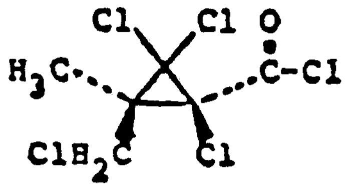

(Verbindung Nr. III-b)

Mittels der gleichen Verfahren wie in den Beispielen a) oder b) wurden die in der Tabelle 1 aufgeführten Verbindungen der allgemeinen Formel (III) gemäß der vorliegenden Erfindung synthetisiert.

**Tabelle 1**

| Verbindung Nr. | Allgemeine Formel (III) | Verfahren |
|---|---|---|
| III-c | 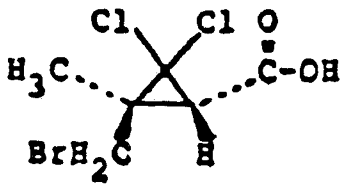 | Beispiel a) |
| III-d | 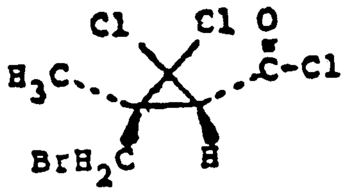 | Beispiel b) |
| III-e | 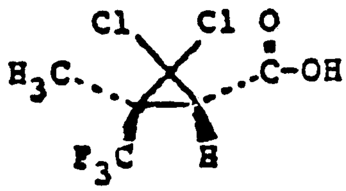 | Beispiel a) |
| III-f | 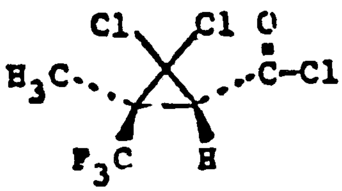 | Beispeil b) |
| III-g | 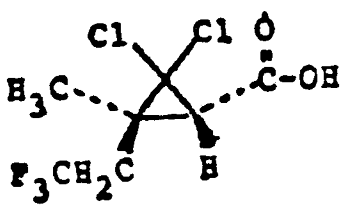 | Beispeil a) |
| III-h | 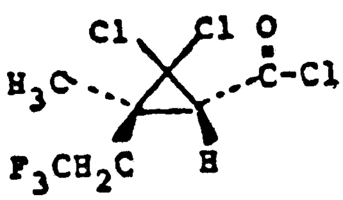 | Beispeil b) |

III-i

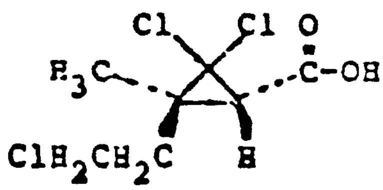

Beispeil a)

III-j

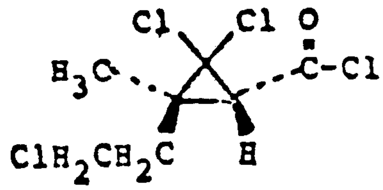

Beispeil b)

Beispiel k)

Methyl-1,2,2-trichloro-3,3-dimethylcyclopropancarboxylat (10 g) wurde in Ameisensäure (20 ml) gelöst, und konzentrierte Schwefelsäure (0,1 g) wurde hinzugefügt. Die Mischung wurde unter leichtem Rückfluß 12 h erhitzt. Nach dem Abkühlen wurde der Reaktionsmischung Wasser (60 ml) zugesetzt. Die ausgefallenen gelben Kristalle wurden durch Filtration gesammelt und aus Hexan umkristallisiert, wonach die gewünschte 1,2,2-Trichloro-3,3-dimethylcyclopropancarbonsäure (3,4 g) der folgenden Formel erhalten wurde; Schmp. 148-150°C.

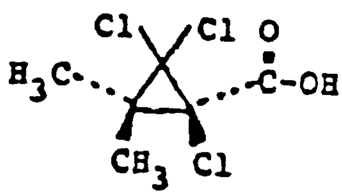

(Verbindung Nr. III-k)

Beispiel 1)

Die in Beispiel k synthetisierte 1,2,2-Trichloro-3,3-dimethylcyclopropancarbonsäure (3,0 g) und Thionylchlorid (1,0 ml) wurden miteinander vermischt und bei 60°C umgesetzt. Die Reaktionsmischung wurde unter vermindertem Druck destilliert, wonach das gewünschte 1,2,2-Trichloro-3,3-dimethylcyclopropancarbonsäurechlorid (2,8 g) der folgenden Formel erhalten wurde.

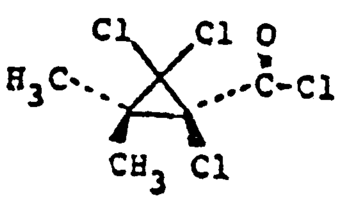

(Verbindung Nr. III-l)

Die in der Tabelle 2 aufgeführten Verbindungen der allgemeinen Formel (III) gemäß der vorliegenden Erfindung wurden mittels der gleichen Verfahren wie in den Beispielen k) oder l) synthetisiert.

**Tabelle 2**

| Verbindung Nr. | Allgemeine Formel (III) | Verfahren |
|---|---|---|
| III-m | 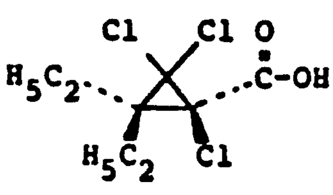 | Beispiel k |

III-n

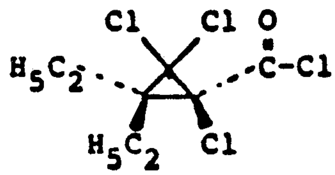

Beispiel l

III-o

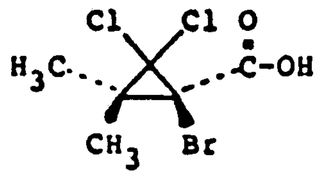

Beispiel k
m. p. 150 - 152°C

III-p

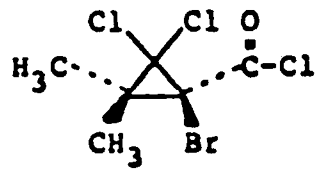

Beispiel l
m. p. 113 - 116°C/20 Torr

III-q

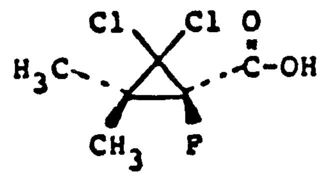

Beispiel k

III-r

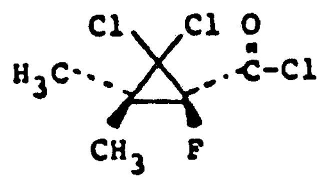

Beispiel l

III-s

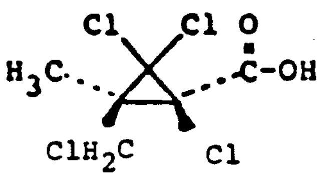

Beispeil k

III-t

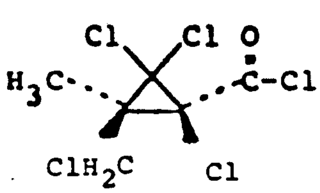

Beispiel l

**Verbindungen der Formel (I)**

Beispiele 1 bis 12

4-Chloro-α-methylbenzylamin (15,6 g) und Triethylamin (10,1 g) wurden in Toluol (150 ml) gelöst. Unter Rühren wurde zu dieser Lösung eine Lösung von 1,2,2-Trichloro-3,3-dimethylcyclopropancarbonsäurechlorid (23,6 g) in Toluol (30 ml) tropfenweise bei 0°C bis 10°C hinzugefügt. Nach der Zugabe wurde die Mischung 2 h

bei Raumtemperatur gerührt. Die Reaktionsmischung wurde in Eiswasser gegossen, und die wäßrige Schicht wurde mit Toluol extrahiert. Die Toluol-Schichten wurden vereinigt, mit einer 1-proz. wäßrigen Salzsäure-Lösung, einer 1-proz. wäßrigen Natriumhydroxid-Lösung und Wasser in dieser Reihenfolge gewaschen und dann über wasserfreiem Natriumsulfat getrocknet. Das Toluol wurde unter vermindertem Druck abgedampft, wonach das gewünschte N-(4-Chloro-α-methylbenzyl)-1,2,2-trichloro-3,3-dimethylcyclopropancarboxamid (32 g) der nachstehenden Formel in Form farbloser Kristalle erhalten wurde; Schmp. 138-139° C.

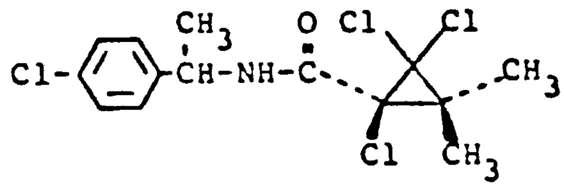

(Verbindung Nr. 1)

Verbindungen der vorliegenden Erfindung, die mit Hilfe des gleichen Verfahrens synthetisiert wurden, wie es im Vorstehenden beschrieben wurde, sind in der folgenden Tabelle 3 aufgeführt.

**Tabelle 3**

| Beispiel Nr. | Verbindung der vorliegenden Erfindung |
|---|---|
| 2 | N-(4-Chlor-α-methylbenzyl)-2,2-dichlor-(trans)-3-chlormethyl-(cis)-3-methylcyclopropancarboxamid (Schmp. 138 - 141° C), |
| 3 | N-(4-Chlor-α-methylbenzyl)-1,2,2-trichlor-3,3-diethylcyclopropancarboxamid, |
| 4 | N-(4-Chlor-α-methylbenzyl-2,2-dichlor-(trans)-3-brommethyl-(cis)-3-methylcyclopropancarboxamid, |
| 5 | N-(4-Chlor-α-methylbenzyl)-2,2-dichlor-(trans)-3-trifluormethyl-(cis)-3-methylcyclopropancarboxamid, |
| 6 | N-(4-Chlor-α-methylbenzyl)-1-brom-2,2-dichlor-3,3-dimethylcyclopropancarboxamid (m. p. 142 - 144° C), |
| 7 | N-(4-Chlor-α-methylbenzyl-2,2-dichlor-1-fluor-3,3-dimethylcyclopropancarboxamid, |
| 8 | N-(4-Fluor-α-methylbenzyl)-1,2,2-trichlor-3,3-dimethylcyclopropancarboxamid, |
| 9 | N-(3,4-Dichlor-α-methylbenzyl)-1,2,2-trichlor-3,3-dimethylcyclopropancarboxamid, |
| 10 | N-(4-Brom-α-methylbenzyl)-1,2,2-trichlor-3,3-dimethylcyclopropancarboxamid, |
| 11 | N-(4-Chlor-α-methylbenzyl)-2,2-dichlor-(trans)-3-(2,2,2-trifluorethyl)-(cis)-3-methylcyclopropancarboxamid, |
| 12 | N-(4-Chlor-α-methylbenzyl)-2,2-dichlor-(trans)-3-(2-chlorethyl)-(cis)-3-methylcyclopropancarboxamid, |
| 13 | N-(4-Chlor-α-methylbenzyl)-1,2,2-trichlor-(trans)-3-chlormethyl-(cis)-3-methylcyclopropancarboxamid. |

**Beispiele 14 bis 27**

4-Chloro-α-methylbenzylamin (15,6 g) und Triethylamin (10,1 g) wurden in Toluol (150 ml) gelöst, und unter Rühren wurde zu dieser Lösung eine Lösung von 2,2-Di-chloro-1-methyl-(trans)-3-methylcyclopropancarbonsäure (20,2 g) in Toluol (30 ml) tropfenweise bei 0°C bis 10°C hinzugefügt. Nach der Zugabe wurde die Mischung 2 h bei Raumtemperatur gerührt. Die Reaktionsmischung wurde in Eiswasser gegossen, und die wäßrige Schicht wurde mit Toluol extrahiert. Die Toluol-Schichten wurden vereinigt, mit einer 1-proz. wäßrigen Salzsäure-Lösung, einer 1-proz. wäßrigen Natriumhydroxid-Lösung und Wasser in dieser Reihenfolge gewaschen und dann über wasserfreiem Natriumsulfat getrocknet. Das Toluol wurde unter vermindertem Druck abgedampft, wonach N-(4-Chloro-α-methylbenzyl)-2,2-dichloro-1-methyl-(trans)-3-methylcyclopropancarboxamid (29 g) der nachstehenden Formel in Form farbloser Kristalle erhalten wurde; Schmp. 132 - 134° C.

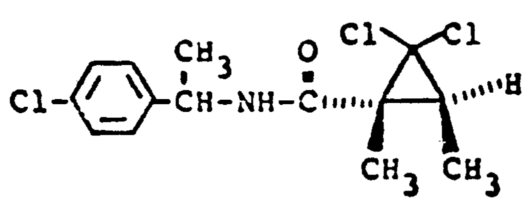

(Verbindung Nr. 14)

Die folgende Tabelle 4 umfaßt die Verbindungen der vorliegenden Erfindung, die nach der für die Verbindung Nr. 14 beschriebenen Arbeitsweise synthetisiert wurden.

**Tabelle 4**

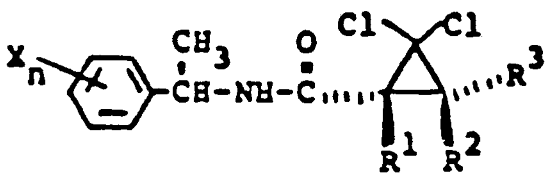

| Verbindung Nr. | $X_n$ | $R^1$ | $R^2$ | $R^3$ | Physikalische Konstante |
|---|---|---|---|---|---|
| 15 | 2-Cl | $-CH_3$ | H | H | Schmp. 161 - 162°C |
| 16 | 4-Cl | $-CH_3$ | H | H | Schmp. 123 - 125°C |
| 17 | 4-Cl | H | $-CH_3$ | H | Schmp. 124 - 126°C |
| 18 | 3,4-$Cl_2$ | $-CH_3$ | H | H | Schmp. 140 - 142°C |
| 19 | 4-Cl | H | $-CH_3$ | $-CH_3$ | Schmp. 132 - 133°C |
| 20 | 4-Cl | $-CH_3$ | $-CH_3$ | $-CH_3$ | Schmp. 147 - 148°C |
| 21 | 4-Cl | $-C_2H_5$ | H | H | Schmp. 135 - 138°C |
| 22 | 4-Cl | H | $-C_2H_5$ | $-C_2H_5$ | |
| 23 | 4-Br | $-CH_3$ | $-CH_3$ | H | Schmp. 126 - 128°C |
| 24 | 4-Cl | $-C_2H_5$ | $-C_2H_5$ | H | |
| 25 | 4-Cl | $-CH_3$ | $-C_3H_7$-n | H | |
| 26 | 4-Cl | H | $-C_4H_9$-n | H | |
| 27 | 4-Br | $-C_2H_5$ | $-C_2H_5$ | $-C_2H_5$ | |
| 28 | 3,4-$Cl_2$ | $-CH_3$ | $-CH_3$ | H | Schmp. 138 - 139°C |
| 29 | 4-Cl | $-C_3H_7$-n | H | H | Schmp. 131 - 132°C |
| 30 | 4-Cl | $-C_3H_7$-i | H | H | |
| 31 | 4-Cl | $-C_2H_5$ | $-CH_3$ | H | Schmp. 168 - 170°C |
| 32 | 4-F | $-CH_3$ | $-CH_3$ | H | Schmp. 127 - 129°C |

**Bezugs-Synthesebeispiel**

Natriumtrichloracetat (55 g) wurde im Laufe von 5 h bei 100°C bis 120°C zu Methyl-2-chlor-3-methyl-2 -butenoat hinzugegeben. Nach der Zugabe wurde die Mischung 2 h weiter bei der gleichen Temperatur gerührt. Nach dem Abkühlen wurde die Reaktionsmischung mit Ether extrahiert. Die etherische Schicht wurde mit Wasser gewaschen und über wasserfreiem Natriumsulfat getrocknet. Der Ether wurde unter vermindertem Druck abgedampft. Der Rückstand wurde unter vermindertem Druck destilliert, wonach das gewünschte Methyl-1,2, 2-trichlor-3,3-dimethylcyclopropancarboxylat (10,3 g) der folgenden Formel erhalten wurde;
Sdp. 156 - 159°C/30,6 mbar (23 mmHg).

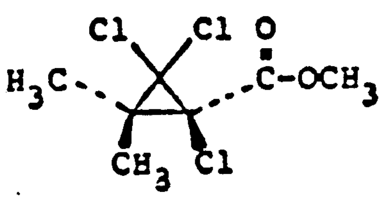

**Formulierungsbeispiele**

Benetzbares Pulver

15 Teile der Verbindung Nr. 1 der Erfindung, 80 Teile eines Gemisches (1 : 5) aus pulvriger Diatomeenerde und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf ein Pathogen und/oder seinen Lebensraum und den Ort, an dem eine Pflanzenkrankheit auftritt, aufgesprüht.

**Emulgierbares Konzentrat**

30 Teile der Verbindung Nr. 1 der Erfindung, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf ein Pathogen und/oder seinen Lebensraum und den Ort, an dem eine Pflanzenkrankheit auftritt, aufgesprüht.

**Stäubemittel**

2 Teile der Verbindung Nr. 3 der Erfindung und 98 Teile Tonpulver werden pulverisiert und gemischt, wodurch ein Stäubemittel hergestellt wird. Dieses wird über einem Pathogen und/oder seinem Lebensraum und dem Ort, an dem eine Pflanzenkrankheit auftritt, ausgestreut.

**Stäubemittel**

Die Verbindung Nr. 1 der Erfindung (1,5 Teile), 0,5 Teile Isopropylhydrogenphosphat (PAP) und 98 Teile Tonpulver werden pulverisiert und gemischt, wodurch ein Stäubemittel hergestellt wird. Dieses wird über einem Pathogen und/oder seinem Lebensraum und dem Ort, an dem eine Pflanzenkrankheit auftritt, ausgestreut.

**Granulat**

Wasser (25 Teile) wird zu einer Mischung aus 10 Teilen der Verbindung Nr. 7 der Erfindung, 30 Teilen Bentonit (Montmorillonit), 58 Teilen Talkum und 2 Teilen Lignosulfonat zugesetzt, und das Gemisch wird gut geknetet. Die Mischung wird mittels eines Extruder-Granulators zu einem Granulat mit einer Korngröße von 0,43 bis 2,0 mm (10 bis 40 mesh) verarbeitet und bei 40°C bis 50°C getrocknet, wodurch ein Granulat hergestellt wird. Das Granulat wird über einem Pathogen und/oder seinem Lebensraum und dem Ort, an dem eine Pflanzenkrankheit auftritt, ausgestreut.

**Granulat**

Ein Drehmischer wird mit 95 Teilen Tonmineral-Teilchen mit einer Teilchengrößen-Verteilung zwischen 0,2 und 2 mm beschickt, und unter Drehen des Mischers werden 5 Teile der Verbindung Nr. 1 der Erfindung gelöst in einem organischen Lösungsmittel auf die Teilchen zur gleichmäßigen Benetzung derselben aufgesprüht, wodurch ein Granulat gebildet wird. Dieses wird dann bei 40°C bis 50°C getrocknet. Das Granulat wird über einem Pathogen und/oder seinem Lebensraum und dem Ort, an dem eine Pflanzenkrankheit auftritt, ausgestreut.

**Biologische Beispiele**

Test der Wirksamkeit gegen die Brusone-Krankheit von Reis durch Anwendung auf das Laub:

**Herstellung eines Test-Präparats:**

| | |
|---|---|
| Aktive Verbindung: | 50 Gew.-% -Teile; |
| Träger: | 45 Gew.-% -Teile einer 1:5-Mischung aus Diatomeenerde und Kaolin; |
| Emulgator: | 5 Gew.-% -Teile Polyoxyethylen-alkylphenylyether. |

Zur Herstellung eines benetzbaren Pulvers werden die aktive Verbindung, der Träger und der Emulgator in den angegebenen Mengen pulverisiert und vermischt. Eine vorher festgelegte Menge des benetzbaren Pulvers wurde mit Wasser verdünnt.

**Test-Verfahren:**

Reispflanzen (Varietät: Asahi) wurden in unglasierten Töpfen mit einem Durchmesser von 12 cm kultiviert. Im 3- bis 4-Blatt-Stadium wurde eine Verdünnung einer Test-Verbindung, die wie oben angegeben mit vorher festgelegter Konzentration hergestellt worden war, auf die Töpfe in einer Aufwandmenge von 50 ml für jeweils 3 Töpfe aufgesprüht. Am nächsten Tage wurde eine Suspension künstlich kultivierter Sporen von Piricularia oryzae zur Inokulierung zweimal auf die Reispflanzen gesprüht. Die Töpfe wurden in einer feuchten Kammer bei einer relativen Luftfeuchtigkeit von 100 % und einer Temperatur von 25°C gehalten, um eine Infektion zu bewirken. Sieben Tage nach der Inokulierung wurde der Grad der Erkrankung nach dem folgenden Standard bewertet, und der Bekämpfungs-Index (%) wurde ebenfalls berechnet.

| Grad der Erkrankung | Verhältnis der Fläche der Verletzungen (%) |
|---|---|
| 0 | 0 |
| 0,5 | 2 oder weniger |
| 1 | 3 bis 5 |
| 2 | 6 bis 10 |
| 3 | 11 bis 20 |
| 4 | 21 bis 40 |
| 5 | 41 oder mehr |

$$\text{Bekämpfungs-Index (\%)} = \frac{\text{Grad der Erkrankung einer unbehandelten Fläche} - \text{Grad der Erkrankung einer behandelten Fläche}}{\text{Grad der Erkrankung einer unbehandelten Fläche}} \times 100$$

Als Ergebnisse sind die Mittelwerte der Einzelergebnisse von jeweils drei Töpfen angegeben, die eine Testfläche bildeten. Die Ergebnisse sind in Tabelle 5 aufgeführt.

**Tabelle 5**

| Beispiel Nr. | Wirkstoff-Konzentration (ppm) | Bekämpfungs-Index (%) |
|---|---|---|
| 1 | 250 | 100 |
| 2 | 250 | 100 |
| 14 | 250 | 100 |
| 16 | 250 | 100 |
| 19 | 250 | 100 |
| 20 | 250 | 100 |
| Vergleich: | | |
| A - 1 | 250 | 0 |
| B - 1 | 250 | 25 |

Anmerkung: Vergleichs-Verbindungen

A - 1:

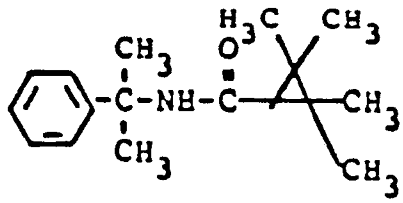

(in der JP-OS 66555/1980 beschriebene Verbindung)

B-1:

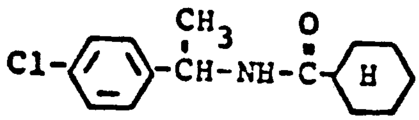

(in der JP-OS 26847/1983 beschriebene Verbindung)

Es wurde nachgewiesen, daß andere Verbindungen gemäß der vorliegenden Erfindung als die in dem vorstehenden Beispiel aufgeführten in dem gleichen Test ungefähr die gleichen Effekte zeigten, wie sie in Tabelle 5 aufgeführt sind.

Test der Rest-Wirksamkeit gegen die Brusone-Krankheit von Reis

**Test-Verfahren:**

Reispflanzen (Varietät: Asahi) wurden in unglasierten Töpfen mit einem Durchmesser von 12 cm kultiviert, und im 3- bis 4-Blatt-Stadium wurde eine Verdünnung einer Test-Verbindung, die wie oben angegeben mit vorher festgelegter Konzentration hergestellt worden war, auf die Töpfe in einer Aufwandmenge von 50 ml für jeweils 3 Töpfe aufgesprüht. Fünf Tage nach dem Sprühen wurden die Reispflanzen zweimal mit einer Suspension künstlich kultivierter Sporen von Piricularia oryzae inokuliert. Die Töpfe wurden in einer feuchten Kammer bei einer relativen Luftfeuchtigkeit von 100 % und einer Temperatur von 25°C gehalten, um eine Infektion der Pflanzen zu bewirken. Sieben Tage nach der Inokulierung wurde der Grad der Erkrankung wie im vorhergehenden Beispiel bewertet, und der Bekämpfungs-Index (%) wurde berechnet. Die Ergebnisse sind in Tabelle 6 aufgeführt.

**Tabelle 6**

| Beispiel Nr. | Wirkstoff-Konzentration (ppm) | Bekämpfungs-Index (%) |
|---|---|---|
| 1 | 250 | 100 |
| | 100 | 100 |
| 6 | 250 | 100 |
| | 100 | 100 |
| 8 | 250 | 100 |
| | 100 | 100 |
| 14 | 500 | 100 |
| | 250 | 100 |
| 16 | 500 | 100 |
| | 250 | 100 |
| 19 | 500 | 100 |
| | 250 | 100 |
| 20 | 500 | 100 |
| | 250 | 100 |

Vergleich:

| | | |
|---|---|---|
| A - 1 | 500 | 0 |
| B - 1 | 250 | 0 |

Anmerkung: Die Vergleichs-Verbindungen sind die gleichen wie in Tabelle 5.

Es wurde nachgewiesen, daß beispielsweise auch die Verbindungen Nr. 10, 23 und 24 gemäß der vorliegenden Erfindung in dem gleichen Test wie vorstehend eine ausgezeichnete Restwirkung zeigten.

Test auf Bekämpfung der Brennfleckenkrankheit der Gurke

**Test-Verfahren:**

Eine Verdünnung einer Test-Verbindung in Form eines wie im Vorstehenden beschrieben hergestellten emulgierbaren Konzentrats wurde in einer vorher festgelegten Konzentration auf in unglasierten 9 cm-Töpfen gezogene Gurken (Varietät: "Suhyo") in einer Menge von 25 ml auf jeweils 3 Töpfe aufgesprüht. Einen Tag nach dem Sprühen wurden die Pflanzen mit einer Sporen-Suspension von Colletotrichum lagenarium inokuliert, und die Pflanzen wurden 1 d in einem Raum mit einer konstanten Temperatur von 23°C und einer Luftfeuchtigkeit von mehr als 90 % stehen gelassen.

Sechs Tage später wurde der Grad der Erkrankung aufgrund des prozentualen Anteils der Läsionen zeigenden Flächen bewertet, und der Bekämpfungsindex (%) wurde berechnet.

| Grad der Erkrankung | Verhältnis der Fläche der Verletzungen (%) |
|---|---|
| 0 | 0 |
| 0,5 | 2 oder weniger |
| 1 | 3 bis 5 |
| 2 | 6 bis 15 |
| 3 | 16 bis 30 |
| 4 | 31 bis 50 |
| 5 | 51 oder mehr |

$$\text{Bekämpfungs-Index (\%)} = \frac{\text{Grad der Erkrankung einer behandelten Fläche}}{\text{Grad der Erkrankung einer unbehandelten Fläche}} \times 100$$

Die Ergebnisse sind in Tabelle 7 aufgeführt.

**Tabelle 7**

| Beispiel Nr. | Wirkstoff-Konzentration (ppm) | Bekämpfungs-Index (%) |
|---|---|---|
| 1 | 500 | 100 |
| 2 | 500 | 100 |
| 14 | 500 | 100 |
| 18 | 500 | 100 |
| Vergleich | | |
| TPN (Handelsprodukt) | 500 | 95 |

Anmerkung:

TPN: Tetrachloroisophthalsäurenitril (75-proz. benetzbares Pulver).

Es wurde nachgewiesen, daß beispielsweise auch die Verbindungen Nr. 12 und 24 gemäß der vorliegenden Erfindung in dem gleichen Test wie vorstehend beschrieben eine ausgezeichnete Bekämpfungswirkung gegenüber der Brennfleckenkrankheit der Gurke zeigten.

## Patentansprüche

1. N-Benzylcyclopropancarboxamid-Derivate der allgemeinen Formel

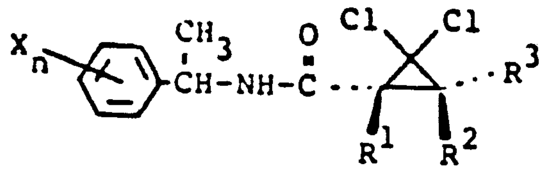

(I)

in der

X Halogen bezeichnet,
n 1 oder 2 bezeichnet,
$R^1$ Wasserstoff, Halogen oder niederes Alkyl bezeichnet,
$R^2$ niederes Alkyl, durch Halogen substituiertes Niederalkyl oder Wasserstoff bezeichnet und
$R^3$ Wasserstoff oder niederes Alkyl bezeichnet.

2. N-Benzylcyclopropancarboxamid-Derivate nach Anspruch 1, dadurch gekennzeichnet, daß in der allgemeinen Formel (I)

X Fluor, Chlor, Brom oder lod bezeichnet,
n 1 oder 2 bezeichnet,
$R^1$ Wasserstoff, Fluor, Chlor, Brom, lod oder Alkyl mit 1 bis 4 Kohlenstoff-Atomen bezeichnet,
$R^2$ Wasserstoff, Alkyl mit 1 bis 4 Kohlenstoff-Atomen und durch Halogen substituiertes Alkyl mit 1 bis 4 Kohlenstoff-Atomen und 1 bis 5 Halogen-Atomen bezeichnet und
$R^3$ Wasserstoff oder Alkyl mit 1 bis 4 Kohlenstoff-Atomen bezeichnet.

3. N-Benzylcyclopropancarboxamid-Derivate nach Anspruch 1, dadurch gekennzeichnet, daß in der allgemeinen Formel (I)

X Fluor, Chlor oder Brom bezeichnet,
n 1 oder 2 bezeichnet,
$R^1$ Wasserstoff, Methyl, Ethyl, n-Propyl, iso-Propyl, Chlor, Brom oder Fluor bezeichnet,
$R^2$ Wasserstoff, Methyl, Ethyl, n-Propyl, iso-Propyl, n-Butyl, tert-Butyl, sec-Butyl, iso-Butyl, Chloromethyl, Bromomethyl, Trifluoromethyl, 2,2,2-Trifluoroethyl oder 2-Chloroethyl bezeichnet und
$R^3$ Wasserstoff, Methyl oder Ethyl bezeichnet.

4. Verfahren zur Herstellung von N-Benzylcyclopropancarboxamid-Derivate der allgemeinen Formel

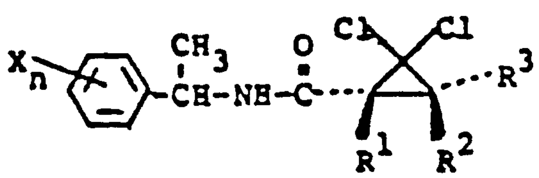

(I)

in der

X Halogen bezeichnet,
n 1 oder 2 bezeichnet,
$R^1$ Wasserstoff, Halogen oder niederes Alkyl bezeichnet,
$R^2$ niederes Alkyl, durch Halogen substituiertes Niederalkyl oder Wasserstoff bezeichnet und
$R^3$ Wasserstoff oder niederes Alkyl bezeichnet, dadurch gekennzeichnet, daß eine Verbindung der allgemeinen Formel

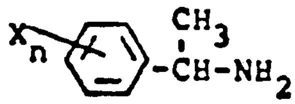

(II)

in der

X und n die im Vorstehenden angegebenen Bedeutungen haben, mit einer Verbindung der allgemeinen Formel

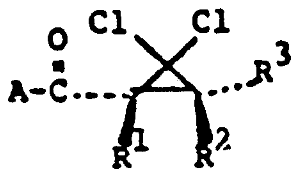

(III)

umgesetzt werden, in der $R^1$, $R^2$ und $R^3$ die im Vorstehenden angegebenen Bedeutungen haben und A ein Hydroxyl oder Halogen bezeichnet.

5. Mittel zur Bekämpfung von Fungi, dadurch gekennzeichnet, daß sie wenigstens ein N-Benzylcyclopropancarboxamid-Derivat der Formel (I) nach den Ansprüchen 1 und 4 enthalten.

6. Verwendung von N-Benzylcyclopropancarboxamid-Derivaten der Formel (I) nach den Ansprüchen 1 und 4 zur Bekämpfung von Fungi.

7. Verfahren zur Bekämpfung von Fungi, dadurch gekennzeichnet, daß man N-Benzylcyclopropancarboxamid-Derivate der Formel (I) nach den Ansprüchen 1 und 4 auf Fungi und/oder ihre Umwelt zur Einwirkung bringt.

8. Verfahren zur Herstellung von Mitteln zur Bekämpfung von Fungi, dadurch gekennzeichnet, daß man N-Benzylcyclopropancarboxamid-Derivate der Formel (I) nach den Ansprüchen 1 und 4 mit Streckmitteln und oder oberflächenaktiven Mitteln vermischt.

**Claims**

1. N-benzylcyclopropanecarboxamide derivatives of the general formula

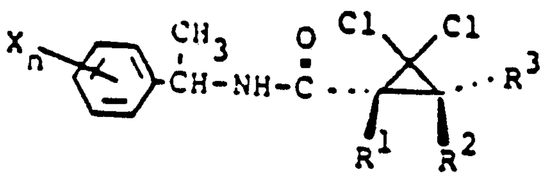

(I)

in which

X represents halogen,
n represents 1 or 2,
$R^1$ represents hydrogen, halogen or lower alkyl,
$R^2$ represents lower alkyl, halogen-substituted lower alkyl or hydrogen and
$R^3$ represents hydrogen or lower alkyl.

2. N-benzylcyclopropanecarboxamide derivatives according to Claim 1, characterized in that, in the general formula (I),

X denotes fluorine, chlorine, bromine or iodine,
n represents 1 or 2,
$R^1$ represents hydrogen, fluorine, chlorine, bromine, iodine or alkyl having 1 to 4 carbon atoms,
$R^2$ represents hydrogen, alkyl having 1 to 4 carbon atoms and halogen-substituted alkyl having 1 to 4 carbon atoms and 1 to 5 halogen atoms and
$R^3$ represents hydrogen or alkyl having 1 to 4 carbon atoms.

3. N-benzylcyclopropanecarboxamide derivatives according to Claim 1, characterized in that, in the general formula (I),

X represents fluorine, chlorine or bromine,
n represents 1 or 2,
$R^1$ represents hydrogen, methyl, ethyl, n-propyl, isopropyl, chlorine, bromine or fluorine,

$R^2$ represents hydrogen, methyl, ethyl, n-propyl, isopropyl, n-butyl, tert-butyl, sec-butyl, isobutyl, chloromethyl, bromomethyl, trifluoromethyl, 2,2,2-trifluoroethyl or 2-chloroethyl and
$R^3$ represents hydrogen, methyl or ethyl.

4. Process for the preparation of N-benzylcyclopropanecarboxamide derivatives of the general formula

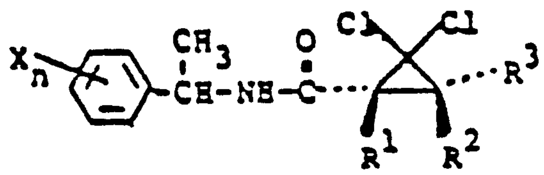
(I)

in which

X represents halogen,
n represents 1 or 2,
$R^1$ represents hydrogen, halogen or lower alkyl,
$R^2$ represents lower alkyl, halogen-substituted lower alkyl or hydrogen and
$R^3$ represents hydrogen or lower alkyl,
characterized in that a compound of the general formula

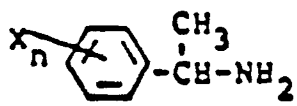
(II)

in which

X and n have the meanings given above are reacted with a compound of the general formula

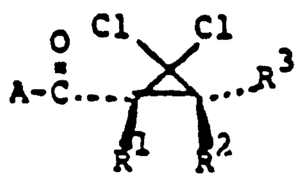
(III)

in which $R^1$, $R^2$ and $R^3$ have the meanings given above and A represents a hydroxyl or halogen.

5. Agents for controlling fungi, characterized in that they contain at least one N-benzylcyclopropanecarboxamide derivative of the formula (I) according to Claims 1 and 4.

6. Use of N-benzylcyclopropanecarboxamide derivatives of the formula (I) according to Claims 1 and 4 for controlling fungi.

7. Method of controlling fungi, characterized in that N-benzylcyclopropanecarboxamide derivatives of the formula (I) according to Claims 1 and 4 are allowed to act on fungi and/or their environment.

8. Process for the preparation of agents for controlling fungi, characterized in that N-benzylcyclopropanecarboxamide derivatives of the formula (I) according to Claims 1 and 4 are mixed with extenders and or surface-active agents.

**Revendications**

1. Dérivés du N-benzylcyclopropane-carboxamide de formule générale

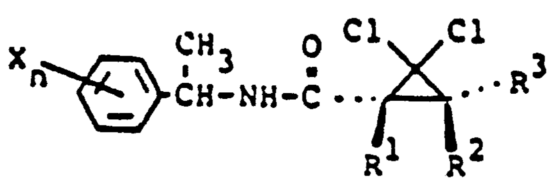
(I)

dans laquelle

X représente un halogène
n est égal à 1 ou 2,

$R^1$ représente l'hydrogène, un halogène ou un groupe alkyle inférieur,

$R^2$ représente un groupe alkyle inférieur, un groupe alkyle inférieur substitué par des halogènes ou l'hydrogène et

$R^3$ représente l'hydrogène ou un groupe alkyle inférieur.

2. Dérivés du N-benzylcyclopropane-carboxamide selon la revendication 1, caractérisés en ce que, dans la formule générale I,

X représente le fluor, le chlore, le brome ou l'iode,

n est égal à 1 ou 2,

$R^1$ représente l'hydrogène, le fluor, le chlore, le brome, l'iode ou un groupe alkyle en $C_1$ - $C_4$,

$R^2$ représente l'hydrogène, un groupe alkyle en $C_1$ - $C_4$ ou un groupe alkyle substitué par des halogènes et contenant 1 à 4 atomes de carbone et 1 à 5 atomes d'halogènes, et

$R^3$ représente l'hydrogène ou un groupe alkyle en $C_1$-$C_4$.

3. Dérivés du N-benzylcyclopropane-carboxamide selon la revendication 1, caractérisés en ce que, dans la formule générale I,

X représente le fluor, le chlore ou le brome,

n est égal à 1 ou 2,

$R^1$ représente l'hydrogène, un groupe méthyle, éthyle, n-propyle, isopropyle, le chlore, le brome ou le fluor,

$R^2$ représente l'hydrogène un groupe méthyle, éthyle, n-propyle, isopropyle, n-butyle, tert-butyle, sec-butyle, isobutyle, chlorométhyle, bromométhyle, trifluorométhyle, 2,2,2-trifluoréthyle ou 2-chloréthyle, et

$R^3$ représente l'hydrogène, un groupe méthyle ou éthyle.

4. Procédé de préparation des dérivés de N-benzylcyclopropane -carboxamide de formule générale

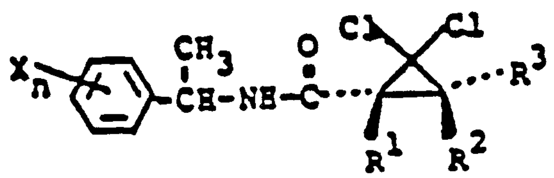

(I)

dans laquelle

X représente un halogène,

n est égal à 1 ou 2,

$R^1$ représente l'hydrogène, un halogène ou un groupe alkyle inférieur,

$R^2$ représente un groupe alkyle inférieur, un groupe alkyle inférieur substitué par des halogènes ou l'hydrogène,

$R^3$ représente l'hydrogène ou un groupe alkyle inférieur, caractérisé en ce que l'on fait réagir un composé de formule générale

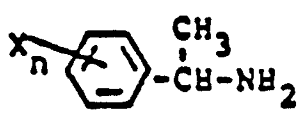

(II)

dans laquelle X et n ont les significations indiquées ci-dessus, avec un composé de formule générale

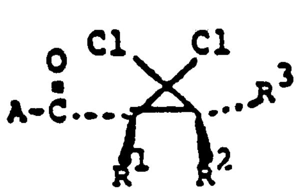

(III)

dans laquelle $R^1$, $R^2$ et $R^3$ ont les significations indiquées ci-dessus et A représente un groupe hydroxy ou un halogène.

5. Produit pour combattre les mycètes, caractérisé en ce qu'il contient au moins un dérivé de N-benzylcyclopropane-carboxamide de formule I selon les revendications 1 et 4.

6. Utilisation des dérivés du N-benzylcyclopropane-carboxamide de formule I selon les revendications 1 et 4, dans la lutte contre les mycètes.

7. Procédé pour combattre les mycètes, caractérisé en ce que l'on fait agir sur les mycètes et/ou leur environnement des dérivés du N-benzylcyclopropane-carboxamide de formule I selon les revendications 1 et 4.

8. Procédé de préparation de produits pour combattre les mycètes, caractérisé en ce que l'on mélange les dérivés du N-benzycylclopropane-carboxamide de formule I selon les revendications 1 et 4 avec des diluants et/ou des agents tensioactifs.